# EUROPEAN PATENT SPECIFICATION

(11) **EP 3 056 099 B1**
(45) Date of publication and mention of the grant of the patent: **04.04.2018**
(21) Application number: 15155269.2
(22) Date of filing: 16.02.2015
(51) Int. Cl.: A24F 47/00

(54) **Electronic smoking device with snap-in locking connection**
Elektronische Rauchvorrichtung mit einschnappbarer Rastverbindung
Dispositif à fumer électronique avec une connexion de verrouillage encliquetable

(43) Date of publication of application: 17.08.2016
(73) Proprietor: Fontem Holdings 1 B.V., 1083 HN Amsterdam (NL)
(72) Inventor: Borkovec, Vaclav, 22761 Hamburg (DE); Wedekind, Ralf, 79254 Oberried (DE); Fröchte, Heinz, 78658 Zimmern (DE); Lambrecht, Andreas, 78048 Villingen-Schwenningen (DE)
(74) Representative: Gulde & Partner

(56) References cited:
- WO-A1-99/20940
- US-A1- 2014 366 898
- US-A1- 2015 027 461

## Description

The present invention relates generally to electronic smoking devices and in particular electronic cigarettes.

An electronic smoking device, such as an electronic cigarette (e-cigarette), typically has a housing containing an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic cigarettes, an airflow sensor is provided within the electronic smoking device which detects a user puffing on the device (e.g., by sensing an under-pressure or an air flow pattern through the device). The airflow sensor indicates or signals the puff to the control electronics to power up the device and generate vapor. In other e-cigarettes, a switch is used to power up the e-cigarette to generate a puff of vapour. US 2015/0027641 A1 is concerned with an electronic cigarette.

Two ways of connecting an atomizer or cartomizer to a battery are generally used.

Most electronic smoking devices use a coaxial screw mechanism to provide the required mechanical and electrical connection between battery and atomizer/cartomizer. This coaxial connection could be incorrectly fitted or cross-threaded, resulting in damage to the device Further the screw thread fitting lacks any indicator to indicate to a user when the atomizer is incorrectly fitted.

An alternative to the screw thread fitting is a 'bayonet' type attachment having protrusions extending outwardly from male fitting which fit into a commentary groove provided on a female fitting. However, this type of fitting is complicated to handle due to the requirement for a push and twist movement. US 2014/0007891 A1 discloses an electronic smoking device according to the preamble of claim 1.

### SUMMARY OF THE INVENTION

According to the invention, an electronic smoking device is provided comprising a main body including a battery; a mouthpiece portion including an atomizer, wherein the main body and the mouthpiece portion are detachable. A first electrical connection element is provided on the main body electrically connected to the battery and a second electrical connection element is provided on the mouthpiece portion electrically connected to the atomizer. One of the first or the second electrical connection elements comprises two connector arms each having a contact portion projecting inwards. The other electrical connection element comprises at least one recess adapted to receive a contact portion such that a snap-in locking connection is provided. The contact portions of the connector arms are spaced apart in a direction along the main axis.

The characteristics, features and advantages of this invention and the manner in which they are obtained as described above, will become more apparent and be more clearly understood in connection with the following description of exemplary embodiments, which are explained with reference to the accompanied drawings.

### BRIEF DESCRIPTION OF THE DRAWINGS

In the drawings, same element numbers indicate same elements in each of the views:
- Figure 1: is a schematic cross-sectional illustration of an exemplary e-cigarette.
- Figure 2: is a side view of an exemplary electrical connector of the atomizer and the battery in an open state.
- Figure 3: is a side view of an exemplary electrical connector of the atomizer and the battery of Figure 2 in a connected state.
- Figure 4: is a schematic perspective view of a further exemplary electrical connector of the atomizer and the battery.
- Figure 5: is a front view of the exemplary electrical connector of Figure 4 in an open state.
- Figure 6: is a front view of the further electrical connector of Figure 4 in a connected state.

### DETAILED DESCRIPTION OF THE DRAWINGS

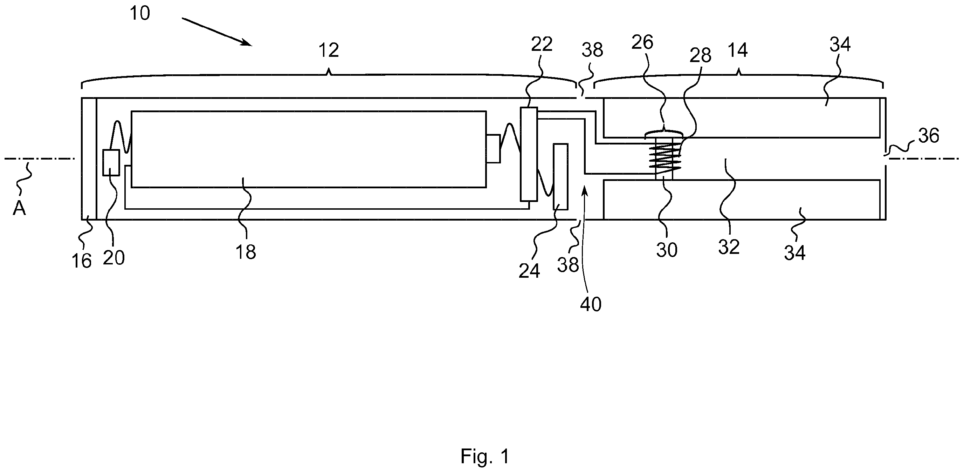

As is shown in Figure 1, an e-cigarette 10 ordinarily comprises a cylindrical housing having a main body 12 and a mouthpiece portion 14. Together the main body 12 and the mouthpiece portion 14 form a cylindrical tube which is approximately the same size and shape as a conventional cigarette, typically about 100 mm with a 7.5 mm diameter, although lengths may range from 70 to 150 or 180 mm, and diameters from 5 to 20 mm. Typically, the outer shape of the e-cigarette 10 is rotationally symmetric with regard to an axis A in the centre of the tube extending along the lateral extent of the e-cigarette 10. The main body 12 and mouthpiece 14 are typically made of steel or hardwearing plastic and act to provide a housing to contain the operative elements of the e-cigarette 10.

An end cap 16 is provided at the end of the main body 12 remote from the mouthpiece portion 14 enclosing that end of the main body 12. The end cap 16 is typically made from translucent plastic material to allow an LED 20 positioned near the end cap to emit light through the end cap. The end cap can be made of metal or other materials that do not allow light to pass. An air inlet may be provided in the end cap, at the edge of the inlet next to the cylindrical hollow tube, anywhere along the length of the cylindrical hollow tube, or at the connection of the battery portion 12 and the atomizer/liquid reservoir portion 14. Figure 1 shows a pair of air inlets 38 provided at the intersection between the battery portion 12 and the atomizer/liquid reservoir portion 14.

A battery 18, a light emitting diode (LED) 20, control electronics 22 and optionally an airflow sensor 24 are provided within the cylindrical hollow tube battery portion 12. The battery 18 is electrically connected to the control electronics 22, which is electrically connected to the LED 20 and the airflow sensor 24. In this example the LED 20 is at the front end of the main body 12, adjacent to the end cap 16 and the control electronics 22 and airflow sensor 24 are provided in the central cavity at the other end of the battery 18 adjacent the atomizer/liquid reservoir portion 14.

The airflow sensor 24 acts as a puff detector, detecting a user puffing or sucking on the mouthpiece portion 14 of the e-cigarette 10. The airflow sensor 24 can be any suitable sensor for detecting changes in airflow or air pressure such a microphone switch including a deformable membrane which is caused to move by variations in air pressure. Alternatively the sensor may be a Hall element or an electro-mechanical sensor.

The control electronics 22 are also connected to an atomizer 26 which in this illustrative example comprises a heating coil 28 which is wrapped around a wick 30 which extends across a central passage 32 provided in the atomizer/liquid reservoir portion 14 of the e-cigarette 10. The coil may be positioned anywhere along the liquid supply and may be positioned transverse or parallel to the liquid reservoir. The dimensions of the central passage 32, the wick 30 and the heating coil 28 are such that the wick 30 and heating coil 28 do not completely block the central passage 32 but rather an air gap is provided either side of the heating coil 28 enabling air to flow past the heating coil 28 and wick 30. The atomizer may alternatively include other forms of heating elements, such as ceramic heaters, or fiber or mesh material heaters. Non resistance heating elements such as sonic, piezo and jet spray may also be used in the atomizer in place of the heating coil.

The central passage 32 is surrounded by a cylindrical liquid supply or store 34 with the ends of the wick 30 abutting or extending into the liquid store 34. The wick 30 comprises a porous material such as a bundle of fiberglass fibers such that liquid present in the liquid store 34 is drawn by capillary action from the ends of the wick 30 towards the central portion of the wick 30 encircled by the heating coil 28.

In some embodiments the liquid store 34 will comprise wadding soaked in liquid which encircles the central passage 32 with the ends of the wick 30 abutting the wadding. In other embodiments the liquid store 34 may comprise a toroidal cavity arranged to be filled with liquid to be vaporized with the toroidal cavity enclosed by walls and with the ends of the wick 30 extending into the toroidal cavity.

An air inhalation port 36 is provided at the back end of the mouthpiece portion 14 remote from main body 12. The inhalation port 36 may be formed from the cylindrical hollow tube atomizer/liquid reservoir portion 14 or maybe formed in an end cap 17.

A pair of air inlets 38 are provided in the housing at the intersection between the main body 12 and the mouthpiece portion 14 adjacent the airflow sensor 24 with the central passage 32 within the mouthpiece portion 14 of the e-cigarette 10 extending from adjacent the air inlets 38 past the wick 30 and heating coil 28 to the air inhalation port 36.

A connector 40 electrically connects the atomizer 26 and the battery 18, enabling the atomizer 26 to be supplied with electric power. Further, the connector 40 allows for a releasable connection of the main body 12 and the mouthpiece portion 14.

In use, a user sucks on the mouthpiece portion 14 of the e-cigarette 10. This causes air to be drawn into the e-cigarette 10 via the one or more air inlets 38 and to be drawn through the central passage 32 towards the air inhalation port 36. The change in air pressure which arises is detected by the airflow sensor 24 which generates an electrical signal that is passed to the control electronics 22. In response to the signal, the control electronics 22 then proceed to activate the heating coil 28 which causes liquid present in the wick 30 to be vaporized creating an aerosol (which may comprise gaseous and liquid components) within the central passage 32. As the user continues to suck on the mouthpiece portion 14 of the e-cigarette 10, this aerosol is drawn up the central passage 32 and inhaled by the user sucking on the e-cigarette 10. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid store 34 by capillary action and thus is available to be converted into an aerosol through subsequent activation of the heating coil 28.

In some e-cigarettes, the e-cigarette 10 is intended to be disposable and the electric power in the battery 18 is intended to be sufficient to vaporize the liquid contained within the liquid store 34 after which the e-cigarette 10 is thrown away. In other embodiments the battery 18 is rechargeable and a means is provided to replenish the liquid supply. In the cases where the liquid store 34 is a toroidal cavity, this may be achieved by providing a refill port and refilling the cavity with liquid via the refill port. In other embodiments the mouthpiece portion 14 of the e-cigarette 10 is detachable from the main body 12 and a new mouthpiece portion 14 can be fitted with a new liquid store 34 thereby replenishing the supply of liquid. In some cases, replacing the liquid store 34 may involve replacement of the heating coil 28 and the wick 30 along with the replacement of the liquid store 34.

In some cases the new liquid store 34 may be in the form of a cartridge. In some such embodiments the cartridge may be provided with a central passage 32 through which a user inhales aerosol generated by the e-cigarette. In other embodiments, rather than inhaling aerosol via a central passage 32, the cartridge may be such to block the central portion of the e-cigarette 10 and generated aerosol may be directed around the exterior of the cartridge 32 to an air inhalation port 36 for inhalation.

It will also be appreciated that although the above description is illustrative of the structure and function of a typical e-cigarette 10, variations also exist. Thus for example in some e-cigarettes the LED 20 is omitted. In some e-cigarettes, the airflow sensor 24 may be placed adjacent the end cap 16 of the e-cigarette rather than in the middle of the e-cigarette as illustrated. Similarly, in some e-cigarettes, the air inlets 36 may be placed at the distal end of the main body 16 of the e-cigarette 10 remote from the mouthpiece portion 14. In some e-cigarettes the airflow sensor 24 is omitted and instead a button is provided which enables a user to activate the e-cigarette manually rather than in response to the detection of a change in air flow or air pressure. Also in some e-cigarettes the constitution of the atomizer may be changed. Thus for example rather than being constituted by a wick 28 surrounded by a heating coil 26 other configurations may be used such as providing a heating coil in a cavity in the interior of a porous body soaked in liquid for atomization and generating an aerosol by evaporating the liquid within the porous body either by virtue of the activation of the coil heating the porous body or alternatively by virtue of the heated air being passed over or through the porous body. Further in some embodiments rather than generating an aerosol through heating liquid within a wick 28 an aerosol might be generated using a piezoelectric atomizer to create an aerosol for inhalation either in combination or in the absence of a heater.

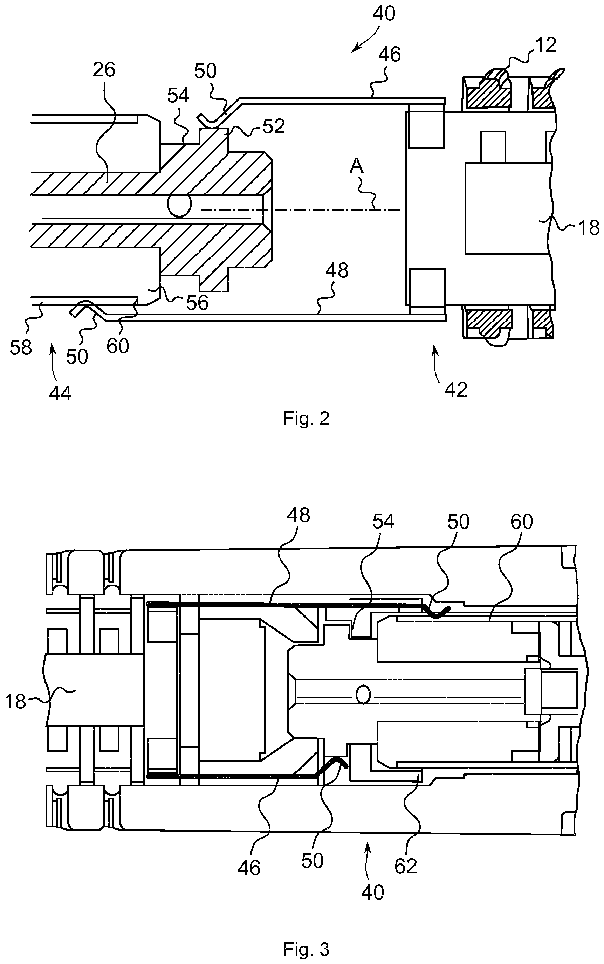

Figure 2 shows an exemplary connector 40 in greater detail. The connector 40 is depicted in a process of connecting the main body 12 and the mouthpiece portion 14 of an e-cigarette 10. The connector 40 has a first electrical connection element 42 which is attached to or part of the main body 12 and a second electrical connection element 44 which is attached to or part of the mouthpiece portion 10. The first electrical connection element 42 is electrically connected to the battery 18.

The first electrical connection element 42 comprises a first connector arm 46 and a second connector arm 48. The two connector arms 46 and 48 extend away from the end of the main body 12 adjacent to the mouthpiece 10 parallel to the axis A running along the centre of the tube formed by the main body 12 and the mouthpiece portion of the e-cigarette 10 with two connector arms 46 and 48 being provided on opposite sides of the axis A. The two connector arms 46 and 48 in this embodiment comprise moulded rigid connectors and may consist for example of spring steel flat bars. Alternatively, at least one of the connector arms 46 and 48 may have the shape of a part of a cylindrical surface. One of the connector arms 46, 48 is a positive contact and connected to the positive battery terminal. The other connector arm is a negative contact and is connected to the negative battery terminal.

At their respective far ends, i.e. distal to the main body 12, the connector arms 46 and 48 have a contact portion 50. The contact portion 50 projects inwards towards the central axis A. In some embodiments the contact portion 50 may have the form of a hook. The contact portions 50 are spaced apart from one another and lie on opposite sides of the axis A. The connector arms 46 and 48 are of different lengths with the first connector arm 46 being shorter than the second connector arm 48. Thus the contact portion 50 of the first connector arm 46 is closer to the main body 12 and the battery 18 and the contact portion of the second connector arm 48 is further from the main body 12 and battery 18.
The second electrical connection element 44 may be partially or totally integrally formed with the atomizer 26. A projection 52 is provided on the atomizer 26 extending away from the mouthpiece 14 with a recess 54 being provided adjacent to the projection 52. The projection 52 is closer to the distal end of the atomizer 26 than the recess 54. In other words the projection 52 lies in front of the recess 54 when seen from the main body 12. The projection 52 and/or the recess 54 may be ring-shaped or in other words rotationally symmetric with regard to the main axis A. Alternatively, the projection 52 and/or the recess 54 may be located only in certain parts of the circumference of the atomizer 26. In that case more than one projection 52 and/or recess 54 may be provided.
Behind the recess 54 an insulator 56 surrounds the inner part of the atomizer 26. At the outer circumference of the insulator 56 a contact area 58 is located. The contact area 58 may comprise a metallic coating or a sleeve of metallic material. As an alternative, the contact area 58 may be formed inside of a recess 60 of the insulator 56. Then, the recess 60 or part of it is coated with a metallic material.
Both recesses 54 and 60 of the second electrical connection element 44 of the atomizer 26 are connected with the heating coil of the atomizer 26 and/or with electronic elements like for example a puff sensor. One of the recesses or contacts 54 and 60 is a positive contact and the other is a negative contact. In a connected state of the electrical connector the contact 50 of the first connector arm 46 is in electrical contact with the recess 54. The contact portion 50 of the second connector arm 48 is in electrical contact with the contact area 58 of the recess 60. The connector 40 is designed such that in a closed state the battery 18 is connected with the coil of the atomizer 26, preferably via a switch or a control device. Electronic components like a puff sensor or a control device may be connected as well.
Beside its electrical functions, the connector 40 connects the main body 12 and the mouthpiece portion 14 in a mechanical way as well. A snap-in locking connection is realized by the connector 40. The snap-in effect is realized by one or two contact portions 50 snapping into the corresponding recess 54 and/or 60. Hence, the connection 40 has a double function. On one hand it serves as the electrical connector between the battery 18 and the atomizer 26 and on the other hand it provides a mechanical connection between the main body 12 and the mouthpiece portion. An advantage of such embodiment may be that it is easy to manufacture and reduces the space needed. Further, it may be easy to use as it provides a much more obvious haptic and sensory feedback that the proper connection has been achieved. It may also be possible to close the connection with only one hand. Closing and opening of the e-cigarette 10 may be very easy and intuitive as only a linear joining movement is necessary.

One or both connector arms 46 and 48 may be biased or bent inwardly, i.e. towards the central axis A of the e-cigarette 10. As an alternative, the connector arms 46 and 48 run parallel to the main axis A and the diameter of the second electrical connection element 44 or the atomizer 26 has a diameter greater than the distance separating the contact portions 50 of the first and second connector arms 46 and 48 so that the connector arms 46 and 48 are bent open by the atomizer 26 when the connection 40 is moved towards a closed position. In more detail, the distance from the main axis A to the outside surface of the projection 52 and/or of the recess 54 is greater than the distance from the main axis A to an inner surface of the contact portion 50 of the first connector arm 46 in an open state of the connector 40. Accordingly, the distance from the main axis A to an outer surface of the contact area 58 of the second electrical connection element 44 is greater than the distance from the main axis A to an inner surface of the contact portion 50 of the second connector arm 48 in an unclosed state of the connection 40.

The strength of the snap-in locking effect may be configured by the radial distances of at least one of the projection 52, the recess 54, the recess 60, the contact portion 50 of the first connector arm 46 and the contact portion 50 of the second connector arm 48. Further, a spring force or elastic force of at least one of the connector arms 46 and 48 can be adjusted to adapt the strength of the snap-in locking effect.

The length of the first and second connector arms 46 and 48, i.e. the axial distance between the two contact portions 50, and the position of the recess 54 and the recess 60 or the contact area 58 on the second electrical connection element 44 are arranged such that the contact portion 50 of the first connector arm 46 co-operates with the recess 54 and/or the projection 52 and that the contact portion 50 of the second connector arm 48 cooperates with the contact area 58 and/or the recess 60. Using these parameters the connection 40 may be configured such that one or both of the connector arms 46 and 48 realize the snap-in locking connection. The electrical connector on the other hand, is always realized by both connector arms 46 and 48.

In the following, some configurations of the mechanical connection are discussed.

The connection 40 in Figure 2 is shown in a half-closed state. The second connector arm has already engaged the contact area 58 inside the recess 60. The contact area 58 comprises an axial length greater than needed for contacting the contact portion 50 of the second connector arm 48. Such length or extension of the contact area 58 provides some clearance for not perfectly matching parts. The contact portion 50 of the first connector arm 46 is in the process of sliding over the projection 52 into the recess 54. After being moved over the projection 52 the contact portion 50 snaps into the recess 54 thereby establishing an electrical and mechanical connection. The distal end of the contact portion 50 may then abut against the insulator 56 which acts as an end stop. Alternatively, an end stop may be provided for the front side of the inner portion of the atomizer 26. As a further alternative, no end stop is provided. In such case, the connector arms 46 and 48 close the connection for example by spring force.

Figure 3 shows the connection 40 in a closed state.

In such case the projection 52 acts as the electrical contact for the contact portion 50 of the first connector arm 46. The connector arms 46 and 48 may then press against the atomizer 26 thereby securing the connection.

As shown in the Figure the contact portion 50 of the first connector arm 46 is in engagement with the projection 52 and the contact portion 50 of the second connector arm 48 is in engagement with the recess 60.

A latch element 62 may be provided for establishing the mechanical snap-in locking connection by engaging the recess 54. The latch element 62 may have a circular shape or may comprise two or more separate parts. The latch element 62 may be part of or attached to the second connector arm 48. The latch element 62 may be defined as a contact portion. With such definition the latch element 62 is a mechanical contact portion (of the second connector arm 48) and the contact portion 50 is the electrical contact portion (of the second connector arm 48).

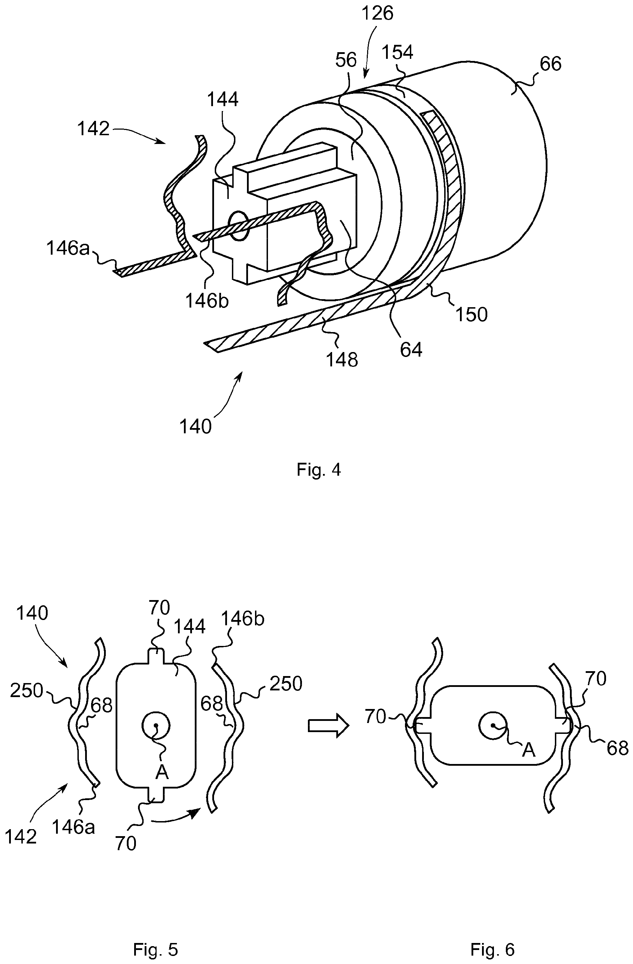

Figures 4 to 6 illustrate a further exemplary electrical connector 140. The electrical connector 140 provides an additional turn-lock connection. Hence, the electrical connector 140 realizes a snap-in locking connection and a turn-lock connection.

The connection 140 includes a first electrical connection element 142 and a second electrical connection element 144. Like in the preceding Figures the first electrical connection element 142 is located at the main body and the second electrical connection element 144 is located at the mouthpiece portion. The second electrical connection element 144 is again formed with the atomizer 126.

The first electrical connection element 142 includes three connector arms, namely two first connector arms 146a and 146b and a second connector arm 148. The second connector arm 148 extends longer than the first connector arms 146a, 146b so that it passes along the inner coaxial part 64 of the atomizer 126 up to an outer coaxial part 66. The inner and outer part is divided by the insulator 56. The outer coaxial part corresponds to a contact area for establishing electrical contact to the second connector arm 148.

The second connector arm 148 includes a contact portion 150 having the shape of a part of a cylindrical surface wherein the radius or curvature is matched to the outer surface of the outer coaxial part 66 of the atomizer 126. At the outer coaxial part 66 of the atomizer 126 a recess 154 is provided for realising a snap-in locking connection. The recess 154 has an annular shape or at least a ring segment shape to allow for relative rotational movement between the atomizer 126 and the second connector arm 148.

The first connector arms 146a and 146b are described in detail in conjunction with Figure 5 and 6. Figure 5 depicts the connection 140 in an open state while Figure 6 depicts the connection 140 in a closed state.

According to Figure 5 the first connector arms 146a and 146b include each a bent contact portion 250 bent partly around the main axis A. Each contact portion 250 has a central indentation 68. The bent contact portion 250 covers a circumference around the main axis A preferably between 90 and 180 degrees. The contact portions 250 and the first connector arms 146a and 146b consist of spring steel or similar electrically conductive and flexible material.
The second electrical connection element 144, here the inner part 64 of the atomizer 126 comprises two opposed protrusions 70. The protrusions 70 and the indentations 68 are adapted to rotationally engage for realizing the turn-lock connection. In order to close the turn locking connection 140 the first electrical connection element 142 and/or the second electrical connection element 144 are turned by preferably 90 degrees. Other angles like for example 45 degrees can also be implemented by moving the indentations 68 out of the middle of the bent contact portions 250. Unlike the bayonet fitting, the turn-lock connection shown in Figures 4 to 6 does not require pushing the components 142 and 144 together to form the connection. Rather, the connection may be made via twisting only, after inserting element 144 into element 142.
During rotation the protrusions 70 press against the contact portions 250 thereby moving or bending them outside, i.e. away from the main axis A. In Figure 6 the closed connection is shown. Then, the protrusions 70 rest inside the indentations 68 thereby defining a strong connection between the second electrical connection element 144 and the first connector arms 146a and 146b. The connection serves as a mechanical and as an electrical connector. For example, the connection between the second electrical connection element 144, i.e. the inner part 64 of the atomizer 126, and the first connector arms 146a and 146b is a negative electrical connector. The connection between the second electrical connection element 144, i.e. the outer part 66 of the atomizer 126, and the second connector arm 148 is the positive electrical connector.
While an embodiment with two first connector arms 146a and 146b has been described a connection including only a single first connector arm 146 is also possible. In such case the necessary counteracting force for the connection is provided by the second connector arm 148 instead of the further first connector arm.
Above, a combined snap-in and turn locking connection has been described. An advantage of this embodiment may be that the connection is very secure due to the two-stage connection.

While the embodiments have been described in the context of an atomizer the invention may also be implemented with a cartomizer, i.e. a combination of an atomizer and a cartridge.
While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.
While in the above described embodiments, a pair of electrical connector arms 46, 48 have been described as extending from the main body 12 of an electronic smoking device 10 and a second electrical connector having protrusions 52 is described as being electrically connected to an atomiser 26, it will be appreciated that the first and second electrical connectors 40, 42 could be interchanged. That is to say that in some embodiments an electrical connector having arms could be provided on the mouthpiece portion 14 of an e- cigarette with a second electrical connector being provided on the main body 12 of the e-cigarette containing a battery 18.

## Claims

1. Electronic smoking device, comprising:
- a main body (12) including a battery (18);
- a mouthpiece portion (14) including an atomizer (26), wherein the main body (12) and the mouthpiece portion (14) are detachable from each other, wherein when attached the main body (12) and the mouthpiece (14) form a shell surrounding a main axis (A);
- a first electrical connection element (42) provided on the main body (12) and electrically connected to the battery (18);
- a second electrical connection element (44) provided on the mouthpiece portion (14) and electrically connected to the atomizer (26);
wherein either the first electrical (42) or the second electrical (44) connection element comprises two connector arms (46, 48) extending parallel to the main axis (A) and each having a contact portion (50) projecting inwards towards the main axis (A) and the other of the first (42) and second electrical connection element (44) comprises at least one recess (54, 60) adapted to receive a contact portion (50) such that a snap-in locking connection (40) is provided **characterized in that** the contact portions (50) of the connector arms (46, 48) are spaced apart in a direction along the main axis (A).

2. Electronic smoking device of claim 1, wherein the connector arms (46, 48) are moulded rigid connectors.

3. Electronic smoking device of claim 1 or 2, wherein the connector arms (46, 48) are arranged on opposite sides of the main axis (A).

4. Electronic smoking device of one of claims 1 to 3, wherein the connector arms (46, 48) consist of spring steel flat bars.

5. Electronic smoking device of one of claims 1 to 4, wherein at least part the second electrical connection element (44) is integrally formed with the atomizer (26).

6. Electronic smoking device of one of claims 1 to 5, wherein one of the first or second electrical connection elements (44) comprises a projection (52) arranged adjacent to the recess (54).

7. Electronic smoking device of one of claims 1 to 6, wherein at least one contact portion (150) has the shape of a part of a cylindrical surface.

8. Electronic smoking device of one of claims 1 to 7, wherein at least one connector arm (46, 48) comprises a bent contact portion (50) bent partly around the main axis (A) with an indentation (68), wherein the other of the first or second connection element (144) comprises a protrusion (70) and wherein the indentation (68) and the protrusion (70) are adapted to rotatory engage for a turn-lock connection.

9. Electronic smoking device of claim 8, wherein the first or the second electrical connection element (142) comprises three connector arms (146a, 146b, 148), wherein two connector arms (146a, 146b) comprise a bent contact portion (250) and wherein the second electrical connection element (144) comprises two protrusions (70).

10. Electronic smoking device of claim 9, wherein the two connector arms (146a, 146b) and the two protrusions (70) are arranged oppositely with regard to the main axis (A).

11. Electronic smoking device of claim 9 or 10, wherein a third of the three connector arms (148) comprises a contact portion (150) having the shape of a part of a cylinder surface adapted for engaging with a recess (154) of the other of the first or second electrical connection element (144) for the snap-in locking connection (40).

## Patentansprüche

1. Elektronische Rauchvorrichtung, umfassend:
- einen Hauptkörper (12), der eine Batterie (18) enthält;
- einen Mundstückteil (14), der einen Zerstäuber (26) enthält, wobei der Hauptkörper (12) und der Mundstückteil (14) voneinander lösbar sind, wobei der Hauptkörper (12) und das Mundstück (14) im aneinander angebrachten Zustand ein Gehäuse ausbilden, die eine Hauptachse (A) umgibt;
- ein erstes elektrisches Verbindungselement (42), das an dem Hauptkörper (12) vorgesehen und elektrisch mit der Batterie (18) verbunden ist;
- ein zweites elektrisches Verbindungselement (44), das an dem Mundstückteil (14) vorgesehen und elektrisch mit dem Zerstäuber (26) verbunden ist;
wobei entweder das erste elektrische (42) oder das zweite elektrische (44) Verbindungselement zwei Verbindungsarme (46, 48) umfasst, die sich parallel zu der Hauptachse (A) erstrecken und jeweils einen Kontaktabschnitt (50) aufweisen, der nach innen, hin zu der Hauptachse (A) vorsteht, und das andere des ersten (42) und zweiten elektrischen Verbindungselements (44) wenigstens eine Aussparung (54, 60) umfasst, die dazu ausgelegt ist, einen Kontaktabschnitt (50) derart aufzunehmen, dass eine einschnappbare Rastverbindung (40) bereitgestellt wird, **dadurch gekennzeichnet, dass** die Kontaktabschnitte (50) der Verbindungsarme (46, 48) in einer Richtung entlang der Hauptachse (A) voneinander beabstandet sind.

2. Elektronische Rauchvorrichtung nach Anspruch 1, wobei die Verbindungsarme (46, 48) geformte starre Verbindungsstücke sind.

3. Elektronische Rauchvorrichtung nach Anspruch 1 oder 2, wobei die Verbindungsarme (46, 48) auf gegenüberliegenden Seiten der Hauptachse (A) angeordnet sind.

4. Elektronische Rauchvorrichtung nach einem der Ansprüche 1 bis 3, wobei die Verbindungsarme (46, 48) aus flachen Federstahlstäben bestehen.

5. Elektronische Rauchvorrichtung nach einem der Ansprüche 1 bis 4, wobei wenigstens ein Teil des zweiten elektrischen Verbindungselements (44) einstückig mit dem Zerstäuber (26) ausgebildet ist.

6. Elektronische Rauchvorrichtung nach einem der Ansprüche 1 bis 5, wobei entweder das erste oder das zweite elektrische Verbindungselement (44) einen Vorsprung (52) umfasst, welcher der Aussparung (54) benachbart angeordnet ist.

7. Elektronische Rauchvorrichtung nach einem der Ansprüche 1 bis 6, wobei wenigstens ein Kontaktabschnitt (150) die Form eines Teils eines Zylindermantels aufweist.

8. Elektronische Rauchvorrichtung nach einem der Ansprüche 1 bis 7, wobei wenigstens ein Verbindungsarm (46, 48) einen teilweise um die Hauptachse (A) herum gebogenen Kontaktabschnitt (50) mit einer Einkerbung (68) umfasst, wobei das andere des ersten oder zweiten Verbindungselements (144) einen Vorsprung (70) umfasst und wobei die Einkerbung (68) und der Vorsprung (70) dazu ausgelegt sind, zwecks einer Drehrastverbindung drehend ineinander einzugreifen.

9. Elektronische Rauchvorrichtung nach Anspruch 8, wobei das erste oder das zweite elektrische Verbindungselement (142) drei Verbindungsarme (146a, 146b, 148) umfasst, wobei zwei Verbindungsarme (146a, 146b) einen gebogenen Kontaktabschnitt (250) umfassen und wobei das zweite elektrische Verbindungselement (144) zwei Vorsprünge (70) umfasst.

10. Elektronische Rauchvorrichtung nach Anspruch 9, wobei die zwei Verbindungsarme (146a, 146b) und die zwei Vorsprünge (70) bezogen auf die Hauptachse (A) gegenüberliegend angeordnet sind.

11. Elektronische Rauchvorrichtung nach Anspruch 9 oder 10, wobei ein dritter der drei Verbindungsarme (148) einen Kontaktabschnitt (150) umfasst, der die Form eines Teils eines Zylindermantels aufweist und zum Ineingriffbringen mit einer Aussparung (154) des anderen des ersten oder zweiten elektrischen Verbindungselements (144) zwecks der einschnappbaren Rastverbindung (40) ausgelegt ist.

## Revendications

1. Dispositif de cigarette électronique comprenant :
- un corps principal (12) incluant une pile (18) ;
- une partie formant embout (14) incluant un nébuliseur (26), le corps principal (12) et la partie formant embout (14) étant séparables l'un de l'autre et le corps principal (12) et l'embout (14), quand rattachés, formant une coque qui entoure un axe principal (A) ;
- un premier élément de connexion électrique (42) situé sur le corps principal (12) et électriquement raccordé à la pile (18) ;
- un deuxième élément de connexion électrique (44) situé sur la portion formant embout (14) et électriquement raccordé au nébuliseur (26) ;
où soit le premier élément de connexion électrique (42) soit le deuxième élément de connexion électrique (44) comprend deux bras connecteurs (46, 48) qui se prolongent parallèlement à l'axe principal (A) et présentent chacun une partie de contact (50) qui se projette vers l'intérieur en direction de l'axe principal (A), l'autre du premier (42) et deuxième éléments de connexion électrique (44) comprenant au moins un évidement (54, 60) adapté pour recevoir une partie de contact (50) de manière à produire une connexion de verrouillage encliquetable (40) **caractérisée en ce que** les parties de contact (50) des bras connecteurs (46, 48) sont espacées l'une de l'autre dans une direction le long de l'axe principal (A).

2. Dispositif de cigarette électronique de la revendication 1, où les bras connecteurs (46, 48) sont des connecteurs moulés rigides.

3. Dispositif de cigarette électronique de la revendication 1 ou 2, où les bras connecteurs (46, 48) sont situés sur des côtés opposés de l'axe principal (A).

4. Dispositif de cigarette électronique de l'une des revendications 1 à 3, où les bras connecteurs (46, 48) consistent en des barres plates en acier à ressorts.

5. Dispositif de cigarette électronique de l'une des revendications 1 à 4, où au moins une partie du deuxième élément de connexion électrique (44) est formé d'une seule pièce avec le nébuliseur (26).

6. Dispositif de cigarette électronique de l'une des revendications 1 à 5, où un parmi le premier et le deuxième éléments de connexion électrique (44) comprend une projection (52) en position adjacente à l'évidement (54).

7. Dispositif de cigarette électronique de l'une des revendications 1 à 6, où au moins une partie de contact (150) a la forme d'un tronçon d'une surface cylindrique.

8. Dispositif de cigarette électronique de l'une des revendications 1 à 7, où au moins un bras connecteur (46, 48) comprend une partie de contact incurvée (50) courbée en partie autour de l'axe principal (A) et dotée d'une entaille (68), où l'autre du premier ou deuxième élément de connexion (144) comprend une saillie (70) et où l'entaille (68) et la saillie (70) sont adaptées pour coopérer en rotation et produire une connexion à verrouillage demi-tour.

9. Dispositif de cigarette électronique de la revendication 8, où le premier ou le deuxième élément de connexion électrique (142) comprend trois bras connecteurs (146a, 146b, 148), deux bras connecteurs (146a, 146b) comprenant une partie de contact incurvée (250) et le deuxième élément de connexion électrique (144) comprenant deux saillies (70).

10. Dispositif de cigarette électronique de la revendication 9, où les deux bras connecteurs (146a, 146b) et les deux saillies sont disposés de part et d'autre de l'axe principal (A).

11. Dispositif de cigarette électronique de la revendication 9 ou 10, où un tiers des trois bras connecteurs (148) comprend une partie de contact (150) ayant la forme d'un tronçon d'une surface cylindre adaptée pour coopérer avec un évidement (154) de l'autre du premier ou du deuxième élément de connexion électrique (144) pour produire la connexion de verrouillage encliquetable (40).
